# NEUE EUROPÄISCHE PATENTSCHRIFT

(11) **EP 0 877 938 B2**
(45) Veröffentlichungstag und Bekanntmachung des Hinweises auf die Entscheidung über den Einspruch: **21.04.2004**
(45) Hinweis auf die Patenterteilung: 10.10.2001
(21) Anmeldenummer: 96946118.5
(22) Anmeldetag: 24.12.1996
(51) Int. Cl.: G01N 33/532, G01N 33/535

(54) **VERFAHREN ZUR MARKIERUNG VON BIOMOLEKÜLEN MIT MEERRETTICHPEROXIDASE**
METHOD OF LABELLING BIOMOLECULES USING HORSERADISH PEROXIDASE
PROCEDE DE MARQUAGE DE BIOMOLECULES AVEC LA PEROXYDASE DU RAIFORT

(30) Priorität: 29.12.1995 DE 19549131
(43) Veröffentlichungstag der Anmeldung: 18.11.1998
(73) Patentinhaber: Biotez Berlin-Buch GmbH, 13125 Berlin (DE)
(72) Erfinder: SCHMIDT, Eberhard, D-13125 Berlin (DE); WEISS, Christa, D-13156 Berlin (DE)
(74) Vertreter: Baumbach, Friedrich
(86) Internationale Anmeldenummer: PCT/DE1996/002511
(87) Internationale Veröffentlichungsnummer: WO 1997/024618

(56) Entgegenhaltungen:
- EP-B- 0 254 172
- EP-B- 0 451 810
- WO-A-84/04970
- WO-A-96/23226
- JOURNAL OF HISTOCHEMISTRY AND CYTOCHEMISTRY, Bd. 22, Nr. 12, 1974, BALTIMORE MD USA, Seiten 1084-1091, XP000671974 P.K. NAKANE ET AL.: "Peroxidase-labeled antibody. A new method of conjugation." in der Anmeldung erwähnt
- W. KNAPP ET AL.: "Immunofluorescence and related staining techniques. " 1978 , ELSEVIER/NORTH HOLLAND BIOMEDICAL PRESS , AMSTERDAM NL XP000672006 in der Anmeldung erwähnt siehe Seite 215, Zeile 1 - Seite 224, Zeile 13
- JOURNAL OF IMMUNOLOGICAL METHODS, Bd. 175, Nr. 1, 1994, AMSTERDAM NL , Seiten 123-129, XP000673598 K. FUJIWARA ET AL.: "New hapten-protein conjugation method using N-(m-aminobenzoyloxy)succinimide as a two-level heterobifunctional agent: thyrotropin-releasing hormone as a model peptide without free amino or carboxyl groups "
- INSERM SYMPOSIUM, Bd. 2, 1976, AMSTERDAM NL, Seiten 7-23, XP000672273 A.J. PESCE ET AL.: "Preparation and analysis of peroxidase antibody and alkiline phasphatase antibody conjugates."

## Beschreibung

Die Erfindung betrifft ein neues Verfahren zur enzymatischen Markierung von Antigenen, wie z. B. Peptiden, Hormonen oder Haptenen, sowie Antikörpern, wie z. B. Immunglobulinen vom IgG- oder IgM-Typ, die als Enzymtracer mit gesteigerter Empfindlichkeit, die der von Radiotracern in Radioimmunoassays (RIA) durchaus ebenbürtig ist, in Enzymimmunoassays eingesetzt werden können. Anwendungsgebiete enzymimmunologischer Nachweisverfahren sind analytische Bestimmungen einer Vielzahl von Inhaltsstoffen im Blut, Harn oder Liquor, die in der Human- und auch Veterinärmedizin für Forschungszwecke, insbesondere jedoch zur praktischen klinischen Diagnostik, breite Anwendung gefunden haben. Ihre Spezifität und Empfindlichkeit hängt in hohem Maße von der Markierungsmethode ab, mit der die Vernetzung des Enzyms mit dem Antigen oder Antikörper vorgenommen wird.

Aus übersichtsarbeiten zum Enzymimmunoassay und verwandter Methoden (EIA) geht hervor, daß eine ganze Reihe von Enzymen bekannt ist, die als Marker geeignet sind *[G. B. Wisdom: Clin. Chem. 22 (1976) 1243 - 1255; A. H. W. M. Schuurs, B. K. van Weemen (1977) in: Enzymimmunoassay, Grundlagen und praktische Anwendung, S. 4 - 9, Georg Thieme Verlag Stuttgart 1978; H. Keller: Medizin. Laboratorium 31 (1978), 83 -* 94; *T. Porstmann, S. T. Kiessig: J. Immunol. Methods 150 (1992) 5 - 21]*. Bis heute ist jedoch Meerrettichperoxidase (HRP) das Enzym der Wahl, was darauf zurückzuführen ist, daß es relativ billig ist, sehr rein und demzufolge mit hoher spezifischer Enzymaktivität hergestellt werden kann und eine "robuste" Biosubstanz ist, mit derchemische Umsetzungen ohne essentiellen Enzymreaktivitätsverlust ausgeführt werden können. Aus diesem Grunde werden in fast 80 % aller kommerziell verfügbaren Enzymimmunoassays HRP-Konjugate verwendet. In den verbleibenden ca. 20 % werden hauptsächlich Konjugate mit alkalischer Phosphatase eingesetzt. Nur in Sonderfällen erfolgt die Markierung mit anderen Enzymen, wie z. B. β-Galaktosidase, Glucoseoxidase oder Acetylcholinesterase u. a.

Bei der Präparation eines Enzymkonjugates besteht die Aufgabe, das Enzym mit der zu markierenden Immunsubstanz durch eine kovalente Bindung zu verknüpfen, ohne daß hierbei weder die Enzymaktivität wesentlich vermindert noch die Immunreaktivität des Antigens oder des Antikörpers beeinträchtigt wird. Das setzt voraus, daß beide Kopplungspartner funktionelle Gruppen besitzen, an denen geeignete Kopplungsreagenzien direkt angreifen können, oder daß zunächst reaktive Gruppen , wie z. B. Thiol- oder Bismaleinimidgruppen eingeführt werden und anschließend in einem zweiten Reaktionsschritt die Konjugation mittels homo- oder heterobifunktioneller Reagenzien auf indirektem Wege erfolgt *[E. Ishikawa et al.: J. Immunoassay* 4 *(1983) 209 - 327]*. Die Bemühungen, derartige H RP-Konjugate als Marker für immunhistochemische Untersuchungen sowie als EIA-Tracer herzustellen, setzten bereits Anfang der 60er Jahre ein. Dem damaligen Entwicklungsstand auf diesem Gebiet entsprechend wurden z. B. 4,4'-Difluor-3,3'-dinitrophenylsulfon *[J. S. Ram: Blochim. Biophys. Acta 78 (1963) 228 -230; P. K. Nakane, G. B. Pierce: J. Histochem. Cytochem. 14 (1966) 929]*, verschiedene Carbodiimide *[S. Avrameas, J. Uriel: C. R. Acad. Sci.{D} Paris* 262 *(1966) 2543; P. K. Nakane, G. B. Pierce: J. Cell Biol. 33 (1967) 307 - 318]*, Cyanurchlorid und bisdiazotiertes o-Dianisidin *[S. Avrameas: Bull. Soc. Chim. Biol. 50 (1968) 1169]* u. a. zur direkten Kopplung von HRP und Immunglobulinen (IgG) verwendet. Als Hauptprodukte dieser sogenannten "Ein-Topf-Verfahren" werden jedoch IgG-Homopolymere gebildet; meist werden nicht mehr als 5 % der eingesetzten HRP in Form des gewünschten HRP-IgG-Konjugats erhalten.

Als Ursache für dieses unerwartete HRP-Reaktionsverhalten stellte sich heraus, daß ein Molekül handelsüblicher Meerrettichperoxidase trotz seines relativ hohen Molekulargewichts von ca. 40000 nur noch ein bis zwei reaktionsfähige Aminogruppen besitzt. Die Mehrzahl der ursprünglich vorhandenen α- und ε-NH₂-Gruppen wird bei Isolierung des Enzyms aus der Pflanze durch Allylisothiocyanat, das sich aus den Inhaltsstoffen Ascorbinsäure und Sinigrin bildet, blockiert *[L. Omstein: J. Histochem. Cytochem. 14 (1966) 929; K. G. Welinder, L. B. Smillie, G. R. Schonbaum: Can. J. Biochem. 50 (1972) 44 - 62]*. Auch bei der 1969 beschriebenen direkten Verknüpfung von HRP mit Immunglobulinen mittels Glutardialdehyd, die unter milden Reaktionsbedingungen über die ε-Aminogruppen des Lysins erfolgt *[S. Avrameas: Immunochemistry 6 (1969) 43],* liegt deshalb die Kopplungsausbeute nur bei 5 - 10 %. Statt dessen kommt es zu einer hochgradigen Selbstverknüpfung des Immunglobulins, wobei hochmolekulare heterogene Konjugate gebildet werden *[A. H. Korn, S. H. Feairheller, E. M. Filachione: J. Mol. Biol. 65 (1972) 525; D. H. Clyne, S. H. Norris, R. R. Modesto et al.: J. Histochem. Cytochem. 21 (1973) 233]*. Es wurde zwar festgestellt, daß die Enzymaktivität nicht wesentlich beeinträchtigt worden ist, aber man fand, daß die Immunreaktivität bei dieser Kopplungsmethode deutlich zurückgegangen ist *[D. M. Boorsma, G. L. Kalsbeck: J. Histochem. Cytochem. 23 (1975) 200]*. Dennoch wurden am Anfang der 70er Jahre zahlreiche HRP-Immunkonjugate für EIA-Zwecke zunächst nach dieser sog. Ein-Schritt-Glutaraldehyd-Methode präpariert.

Bereits im Jahre 1971 gelang es, dieses Ein-Schritt-Kopplungsverfahren mit Glutardialdehyd zu verbessern *[S. Avrameas, T. Ternynck: Immunochemistry 8 (1971) 1175-1179]*, nachdem man erkannt hatte, daß ein HRP-Molekül aufgrund der sehr kleinen Zahl reaktionsfähiger Aminogruppen selbst bei überschuß an Kopplungsreagens nur mit einem Molekül Glutardialdehyd reagieren kann. Die zweite Aldehydgruppe ist offenbar nicht fähig, mit dem gleichen oder einem anderen HPR-Molekül eine Reaktion einzugehen. Dieses eigentümliche Reaktionsverhalten der HRP ist die Grundlage für das sog. Zwei-Schritt-Kopplungsverfahren, bei dem das Enzym zunächst allein mit Glutardialdehyd zur Reaktion gebracht wird. Nach Abtrennung des überschusses an Kopplungsreagens erhält man ein monomeres HRP-Glutardialdehyd-Kopplungsprodukt, quasi eine "aktivierte Peroxidase", die imstande ist, in einem zweiten Reaktionsschritt mit primären NH₂-Gruppen eines Antigens oder Antikörpers zu reagieren. Hierbei werden vorzugsweise momomere Konjugate gebildet *[S. Avrameas: Histochem. J. 4 (1972) 321]*. Es entstehen jedoch auch Konjugate, die 2 Mole Enzym pro IgG-Molekül enthalten *[D. M. Boorsma, J. G. Streefkerk: J. Immunol. Methods 30 (1979) 245 - 255]*. Die HRP-Reaktivität wird auch bei dieser Konjugationsmethode um 30 - 50 % herabgesetzt, der Verlust an Immunreaktivität ist jedoch geringer als bei der Ein-Schritt-Methode *[T. J. Greenwalt, E. McF.Swierk, E. A. Steaner: J. Histochem. Cytochem. 21 (1973) 233]*, die Effektivität der Enzymverknüpfung ist etwas höher *[N. Yamamoto: Acta Histochem. Cytochem. 8 (1975) 41]*. Ungeachtet der angeführten Nachteile wurden nach dieser Methode zahlreiche HRP-Konjugate zur Anwendung als EIA-Tracer hergestellt *[B. K. Weeman, A. H. W. M. Schuurs: FEBS Lett. 15 (19771) 232: S. Avrameas, B. Guilbert: Biochimie 54 (1972) 837]*.

Der trotz steigender Anwendung enzymimmunologischer Nachweisverfahren zu Beginn der 70er Jahre keinesfalls zufriedenstellende Stand bei der Präparation der hierzu erforderlichen Enzymkonjugate war möglicherweise der Anlaß, daß NAKANE und KAWOI 1974 eine völlig neue Strategie zur Präparation HRP-markierter Antikörper entwickelten. Sie gingen davon aus, daß es sich bei den meisten Enzymen um Glykoproteine handelt. Bei der Meerrettichperoxidase beträgt der Kahlenhydratanteil etwa 18 % seines Molekulargewichtes und besteht aus B Kohlenhydratketten bekannter Zusammensetzung, die an der Oberfläche des Enzyms angeordnet sind, jedoch in keiner Beziehung zur Enzymaktivität des Moleküls stehen *[L. Shannon, E. Kay, J. Y. Lew: J. Biol. Chem. 241 (1966) 2166 - 2172; K. G. Welinder, L. B. Smillie: Can. J. Biochem. 50 (1972) 63 - 90; K. G. Welinder: Eur. J. Biochem. 96 (1979) 483 - 509]*. Wird Meerrettichperoxidase mit Natriummetaperjodat oxydiert, werden die Kohlenhydratreste mit vicinalen OH-Gruppen gespalten und Aldehydgruppen gebildet, ohne daß es hierbei zu einem signifikanten Verlust an Ehzymaktivität kommt *[P. K. Nakane, A. Kawoi: J. Histochem. Cytochem. 22 (1974) 1084 - 1091]*. Auf diese Weise entsteht wiederum eine "aktivierte" HRP, die mit primären NH₂-Gruppen eines Antigens oder Antikörpers unter Bildung Schiff'scher Basen direkt reagieren. kann. Zweckmäßigerweise wird die ungesättigte Azomethin-Bindung anschließend durch Hydrierung mit Natriumborhydrid stabilisiert. Die Abtrennung des HRP-markierten Tracers wird in bekannter Weise durch Gelfiltration oder Dialyse vorgenommen.

Durch das von NAKANE eingeführte Kopplungsverfahren werden die Schwierigkeiten ausgeschaltet, die sich bei den bisher praktizierten Konjugationsmethoden dadurch ergaben, daß ein HRP-Molekül im Vergleich zu einem IgG-Molekül nur ein Fünfzigstel oder sogar weniger reaktive primäre Aminogruppen besitzt, an denen die verwendeten Kopplungsreagenzien angreifen können. Da die IgG-Selbstverknüpfung entfällt, ist die Effektivität der Kopplung hoch: sie beträgt etwa 70 % bezüglich HRP und 90 - 95 % bezüglich IgG. Auch bei dieser Methode bleiben weder die Enzymaktivität noch die Immunreaktivität völlig erhalten. Die Angaben, die man hierüber in der Literatur findet, sind unterschiedlich, sie stehen in engem Zusammenhang mit den Reaktionsbedingungen, unter denen die Natriummetaperjodat-Oxydation sowie die Reduktion mit NaBH₄ ausgeführt wird. Dadurch, daß das oxydierte HRP-Molekül eine größere Zahl Glucosehemiglutaryl-Linker an seiner Oberfläche besitzt, ist es in der Lage, nicht nur mit einem, sondern mit mehreren IgG-Molekülen zu konjugieren. Je nachdem, in welchem stöchiometrischen Verhältnis oxydierte HRP und IgG umgesetzt werden, kommt es zur Bildung vernetzter Aggregate, deren Molekulargewicht 400000 und mehr erreichen kann. Um eine Verknüpfung der HRP-Moleküle untereinander auszuschließen, wurden in den Anfangsjahren die wenigen noch im HRP-Molekül vorhandenen primären Aminogruppen vor der NalO₄-Oxydation mit 1-Fluor-2,4-dinitro-benzol blockiert.

Nach eingehenden Untersuchungen darüber, welchen Einfluß die einzelnen Stufen der NalO₄-Kopplungsmethode sowohl auf die enzymatischen und auch die immunologischen Eigenschaften der HRP-IgG-Konjugate ausüben, publizierten WILSON und NAKANE im Jahre 1978 ein verbessertes Verfahren, das bis heute die Grundlage für die Peroxidasemarkierung darstellt *[B. Wilson, P, K. Nakane: Recent Developments in the Periodate Method of Conjugating Horseradish Peroxidase to Antibodies. In: Immunofluorescence and Related Staining Techniques (Eds.: W. Knapp, K. Holubar, G. Wick), pp 215 - 224, Elsevier*/-*North-Holland Biomedical Press 1978]*. Als wesentliche Verbesserung wird angeführt, daß die früher vor der NalO₄-Oxydation der HRP empfohlene Blokkierung der primären NH₂-Gruppen mit 1-Fluor-2,4-dinitrobenzol entfallen kann. Es hatsich nämlich gezeigt, daßtrotz dieser Vorsichtsmaßnatime die Selbstverknüpfung der oxydierten HRP nicht vollständig unterdrückt werden kann, da die Schutzblockierung in alkalischer Pufferlösung erfolgen muß. In alkalischem Milieu kommt es überdies auch zu einer partiellen Inaktivierung des Natriummetaper jodats. Es ist deshalb günstiger, wenn die Oxydation der HRP in neutraler bzw. schwach essigsaurer Lösung ausgeführt wird. Auch die Abtrennung des Perjodatüberschusses durch Dialyse oder Gelfiltration wird deshalb bei einem ph-Wert zwischen 4 und 5 vorgenommen. Selbst bei kurzfristiger Lagerung der durch die gebildeten Aldehydgruppen aktivierten HRP bei diesem niedrigen pH-Wert bleibt die Selbstkonjugation unter 5 %. Optimale Reaktionsbedingungen liegen vor, wenn die 1. Stufe des WILSON-NAKANE-Kopplungsverfahrens, die HRP-Oxydation, bei einer NalO₄-Konnzentration ≤ 0,02 M bei Raumtemperatur und einem 200fachem molarem überschuß bezüglich Peroxidase im Dunkeln ausgeführt wird. Auf diese Weise wird vermieden, daß es zu einer fotochemischen Oxydation der Kohlenhydratreste durch Ozon kommt, das bei der Zersetzung des NalO₄ im Licht gebildet wird *[E. B. Dikowa, E.* *M, Gawrilowa, A. M. Jegorow: Bioorgan. Khim. [Moskau] 16 (1990) 476 - 481]*. Eine Reaktionszeit von 20 Minuten reicht aus; es gibt Arbeitsgruppen, die sogar auf 10 Minuten verkürzen *[M. Imagawa*, S. *Yoshitake, Y. Hamaguchi et al.: J. appl. Biochemistry 4 (1982) 41 - 57]*. WILSON und NAKANE geben an, daß ein auf diese Weise mit NalO₄ oxydiertes HRP-Molekül mindestens 18 reaktive Aldehyd-Fängergruppen, an seiner Oberfläche besitzt. Vermutlich aus sterischen Gründen können allerdings nicht alle zur Kopplung mit IgG-Molekülen ausgenützt werden.

Die 2. Stufe des Kopplungsverfahrens, die Konjugation der oxydierten HRP mit dem Immunglobulin oder anderen Proteinen, die mit Meerrettichperoxidase markiert werden sollen, wird in bicarbonatgepufferter Lösung bei pH ≥ 9, optimal zwischen 9,5 und 9,8, unter Bildung Schiff' scher Basen ausgeführt. WILSON und NAKANE geben an, daß die Kopplungszeit nicht unter 2 Stunden bei Raumtemperatur liegen sollte. Günstiger ist es, wenn man 4 Stunden bei Raumtemperatur reagieren läßt. In Abhängigkeit vom stöchiometrischen Verhältnis der Reaktionspartner werden hierbei Vielkomponentengemische von Oligomeren unterschiedlicher Molekularmasse gebildet. Wird die oxydierte HRP in deutlichem überschuß eingesetzt, können 5 - 6 HRP-Moleküle ohne sterische Behinderung an ein IgG-Molekül gebunden werden. Da ein oxydiertes HRP-Molekül vermittels seiner Aldehydgruppen wiederum mit mehr als einem IgG-Molekül reagieren kann, ist die Bildung polymerer Konjugate unvermeidlich. Ein Modell, das dieses komplexe Reaktionsgeschehen bei Reaktionsverhältnissen bis zu 1,0 in guter Übereinstimmung mit den von WILSON und NAKANE gefundenen experimentellen Ergebnissen beschreibt, stammt von ARCHER *[P G. Archer: Biometrics 32 (1976) 369 - 375]*. Bei höheren Reaktionsverhältnissen liegen die experimentellen unter den modellgemäß berechneten Werten, was auf sterische Einflüsse zurückgeführt wird. Diese machen sich auch bei der spektralfotometrischen Bestimmung des Peroxidaseanteils eines Konjugats, der bei 403 nm gemessen wird, und seinem Gesamtproteingehalt, der sich aus der Extinktion bei 280 nm ergibt und fehlerhaft wesentlich zu hoch ausfällt, störend bemerkbar.

Ein kritischer Schritt ist zweifellos die 3. Stufe des WILSON-NAKANE-Kopplungsverfahrens, die Hydrierung mit Natriumborhydrid, um die ungesättigten Azomethinbindungen der Schiff'schen Basen zu stabilisieren und gleichzeitig die überschüssigen Aldehydgruppen durch Reduktion in die entsprechenden Carbinole zu überführen. Diese Reaktion erfolgt unmittelbar im Anschluß an die Kopplung. Sie wird bei pH 9,5 in bicarbonatgepufferter Lösung bei 4 °C ausgeführt, wobei NaBH₄ in 100fachem molarem überschuß in bezug auf eingesetzte HRP angewendet wird. Die Reaktionszeit sollte nicht unter 2 Stunden liegen. Obwohl NaBH₄ ein selektives Reduktionsmittel ist, wird von verschiedenen Arbeitsgruppen angegeben, daß seine Anwendung zu einem merklichen Verlust an Enzymaktivität führt, der 18 % und mehr betragen kann. Aus diesem Grunde empfiehlt die amerikanische Firma PIERCE, auf die Stabilisierung der Azomethinbindungen zu verzichten und lediglich die überschüssigen Aldehydgruppen mit Lysin oder Ethanolamin zu quenchen *[PIERCE Immuno-Technology, Catalog& Handbook 1992]*, oder statt NaBH₄ das weniger starke Reduktionsmittel Natriumcyanborhydrid zu verwenden, das auch gegenüber IgG eine bessere Kompatibilität aufweisen soll *[PIERCE Seminar 5: Antibody-Enzyme Conjugates, Methods for Preparation and Puritication 1993]*.

Die letzte Stufe des WILSON-NAKANE-Kopplungsverfahrens besteht in der Isolation des hochmolekularen Konjugats aus dem Kopplungsgemisch. Die Abtrennung niedermolekularer Kopplungsprodukte, nicht HRP-markierten Ausgangsproteins sowie des freien Enzyms ist ein ganz wesentlicher Schritt, um einen HRP-Tracer mit optimalen Eigenschaften zu erhalten, und führt zu einer erheblichen Steigerung der Sensitivität des Assays. Diese Trennaufgabe kann weder durch Dialyse noch durch Gelfiltration und Abtrennung des Tracers im Ausschlußvolumen einer mit Sephadex G-25 gefüllten Säule gelöstwerden, sondern erfolgt durch Gelpermeationschromatografie übereine Sephacryl-5-300-Säule (Pharmacia), die mit 0,05 M PBS-Puffer (0,15 M NaCl) vom pH-Wert 7,4 eluiert wird.

Zusammenfassend wird festgestellt, daß die Präparation eines HRP-IgG-Konjugates nach dem WILSON-NAKANE-Verfahren zu einem hochmolekularem Komplex führt, in dem das Immunglobulinmolekül quasi den Kern darstellt, der kovalent an seiner Oberfläche so viele HRP-Moleküle über ihre aktivierten Aldehydgruppen binden kann, wie es die sterischen Verhältnisse gestatten. Eine Verknüpfung der HRP-Moleküle untereinander wird vermutlich nur in untergeordnetem Maße eintreten. Sensitive HRP-Tracer für EIA-Anwendung bedingen jedoch, daß der HRP-Substitutionsgrad nicht unter 2, sondern besser deutlich höher liegen sollte. Mit steigendem Substitutionsgrad wird allerdings die Oberfläche des zentralen Immunglobulins mehr und mehr abgeschirmt. Das ist möglicherweise zumindest teilweise die Erklärung für die beobachtete Verminderung der immunologischen Reaktivität. Auch die spektralfotometrisch bei 280 nm gefundenen fehlerhaften Meßwerte für den Gesamtproteingehalt könnten ihre Ursache in der beschriebenen Struktur des HRP-Konjugate haben.

Das Ziel der Erfindung ist es, das von MILSON und NAKANE eingeführte Verfahren zur Markierung von Biomolekülen mit Meerrettichperoxidase so zu verbessern, daß strukturell modifizierte HRP-Protein-Konjugate erhalten werden, in denen sich weder die enzymatischen Bereiche nach die für das immunologische Reaktionsvermögen verantwortlichen Bindungsstellen des Gesamtmoleküls aufgrund sterischer Behinderung gegenseitig beeinflussen. Das sollte sich auch bei der Mehrzahl der präparierten HRP-Konjugate auf die Ergebnisse der spektralfotometrischen Messung, neben der bei 402 nm für das Enzym bestimmbaren Extinktion insbesondere für die bei 278 nm, die den Gesamtproteingehalt angibt, auswirken und damit eine zuverlässigere analytische Berechnung möglich machen.

Der Erfindung liegt die Aufgabe zugrunde, ein auf der Methode von WILSON und NAKANE aufbauendes, neues Verfahren zur enzymatischen Markierung von Biomolekülen verschiedenster Art, vorzugsweise von Immunglobulinen, zu schaffen nach dem HRP-Tracer mit einer Testsensivität hergestellt werden können, die bisher nur in RIA's erreicht worden sind. Darüber hinaus sollen auch die spektralfotometrischen Meßprobleme, die eine analytische Charakterisierung der Präparate bisher einschränkten, weitgehend zurückgedrängt werden.
Die Erfindung wird gemäß Anspruch 1 dadurch realisiert, daß nach der Perjodatoxydation der nativen Meerettichperoxidase, die im wesentlichen den Angaben von WILSON und NAKANE entsprechend ausgeführt wird und bei der an jedem HRP-Molekül eine so große Zahl von Aldehydgruppen erzeugt wird, die ohnehin bei der anschließenden direkten Kopplung eines größeren Proteinmoleküls wie beispielsweise IgG nicht ausgenutzt werden kann, zunächst eine intermolekulare Vernetzung der HRP-Moleküle mit einem längerkettigen α,ω-Diamino-oxa-alkan vorgenommen wird. Die Strukturierung eines derartigen HRP-Netzes ist davon abhängig, welche Länge der verwendete bifunktionelle Spacer aufweist und in welchem stöchiometrischen Verhältnis er mit der oxydierten Meerettichperoxidase zur Reaktion gebracht wird.

Für die Peroxidase-Vernetzung besonders gut geeignet sind längerkettige α,ω-Diamino-oxa-alkane, in deren Kette mehrere Oxa-Brücken enthalten sind. Derartige Verbindungen sind Flüssigkeiten, die unbegrenzt mit Wasser mischbar sind. Auf diese Weise kann die Spacerlänge wesentlich erhöht werden, wodurch das HRP-Netz aufgelockert wird, ohne daß die Wasserlöslichkeit beeinträchtigt wird. Das erst seit kurzer Zeit im Handel erhältliche 1,13-Diamino-4,7,10-trioxa-tridecan (DTT) weist sehr günstige Eigenschaften für die Peroxidase-Vernetzung auf. Im Anhang sind die für die Peroxidase-Vernetzung geeigneten α,ω-Diamino-oxa-alkane in einer Übersichtstabelle zusammengefaßt.

Die Vernetzung selbst wird zweckmäßigerweise so ausgeführt, daß nur ein kleiner Teil der bei der Perjodatoxydation gebildeten Aldehydgruppen verbraucht wird. Mehr als einen 2,5-bis 3,5fachen molaren Überschuß an DTT in Bezug auf eingesetzte HRP sollte man nicht wählen. Man erhält dann ein hochmolekulares HRP-Konjugat mit außerordentlich hoher Enzymaktivität. Mit der durch Vernetzung bewirkten Molekülvergrößerung steigt jedoch auch die unspezifische Bindung eines solchen HRP-Tracers im EIA an. Um diese in vertretbaren Grenzen zu halten, ist es günstiger, das α,ω-Diamino-oxa-alkan nur in 0,5- bis 1,5fachem molaren Überschuß zum Zwecke der Vernetzung mit HRP zur Reaktion zu bringen. Wird zur Vernetzung mehr als der 3,5fache molare Überschuß an DTT eingesetzt, steigt der nicht an das HRP-Netz gekoppelte Proteinanteil, und die Ausbeute an HRP-Konjugat geht zurück.

Zur Vernetzung wird eine 0,002 M Lösung des HMD bzw. DTT in 0,1 M Bicarbonatpuffer (pH 9,8) hergestellt, die längerfristig verwendbar ist. 75µl dieser Lösung werden zu der nach Perjodatoxydation von 4 mg nativer Meerettichperoxidase in 0,005M Acetatpuffer (pH 4,3) vorliegenden HRP (ox.)-Lösung, die durch Ultrafiltration auf ein Volumen von ca. 0,2 ml konzentriert worden ist, zugefügt. Zur Vernetzung ist eine Reaktionszeit von 4, Stunden bei Raumtemperatur erforderlich; sie kann auch über Nacht im Kühlschrank bei 4°C ausgeführt werden.

Dadurch wird ein Aggregat erzeugt, das aus 6 bis 10 HRP-Molekülen besteht, an das ohne vorherige Abtrennung aus dem Reaktionsgemisch über die noch vorhandene Aldehydgruppen ein oder auch mehrere Immunglobulinmoleküle bzw. auch andere Proteine kovalent gebunden werden können. Praktisch wird das dadurch verwirklicht, daß das in 0,1 M Bicarbonatpuffer (pH 9,8) gelöste Protein zur vernetzten HRP-Lösung oder umgekehrt gegeben wird. Die Reaktionsbedingungen sind die gleichen wie bei der Vernetzung, die Reaktion wird also nur weitergeführt. Auf diese Weise wird ein wesentlich höherer Substitutionsgrad erreicht. Da das IgG-Molekül nicht durch HRP-Moleküle abgeschirmt wird, kann es seine immunologischen Eigenschaften ohne Einschränkung entfalten und ist spektralfotometrisch fehlerfrei meßbar. Selbstverständlich müssen die ungesättigten Azomethinbindungen auch bei diesem Verfahren durch Hydrierung mit Natriumborhydrid oder Natriumcyanborhydrid stabilisiert werden. Wird die Separation des HRP-markierten Tracers durch Gelpermeations-Chromatografie über Sephacryl S-300 vorgenommen, wodurch es möglich ist, im Reaktionsgemisch vorhandene niedermolekulare Kopplungsprodukte, gegebenenfalls nicht gekoppeltes Protein sowie auch nicht vernetzte, also freie HRP abzutrennen, zeichnet sich das Präparat durch optimale Eigenschaften aus.

Insgesamt stellt das neue HRP-Markierungsverfahren einen bemerkenswerten Fortschritt für die Entwicklung leistungsstarker enzymimmunologischer Nachweissysteme dar. Statt einer ungesteuerten Bildung heterogener Kopplungsprodukte wird durch das erfindungsgemäße Verfahren die Meerettichperoxidase nach Oxydation mit Natriummetaperjodat zunächst in gezielter Weise mit einem α,ω Diamino-oxa-alkan, vorzugsweise 1,13-Diamino-4,7,10-trioxatridecan, vernetzt, bevor die Kopplung mit dem zu markierenden Protein erfolgt. Dadurch kann ein hoher Substitutionsgrad erreicht werden, ohne daß die enzymatischen und insbesondere die immunologischen Eigenschaften wesentlich verändert werden. Die Empfindlichkeit und Spezifität der erhaltenen HRP-Tracer ist außerordentlich hoch und Radiotracern durchaus vergleichbar. Ihre analytische Charakterisierung auf der Grundlage spektralfotometrischer Messungen liefert zuverlässige Werte, wenn die bei 280 nm gemessene Extinktion für das Protein korrigiert wird, indem die auf HRP bei dieser Wellenlänge entfallende konzentrationsabhängige Extinktion berücksichtigt wird.

### AUSFÜHRUNGSBEISPIEL

Zur Markierung eines Immunglobulins (IgG) mit Meerrettichperoxidase (HRP) sind folgende 5 Präparationsschritte auszuführen:
1. HRP-Oxydation mit NalO₄
2. HRP(ox.)-Vernetzung mit 1,13-Diamino-4,7,10-trioxa-tridecan (DTT)
3. IgG-Kopplung mit vernetzter HRP
4. Stabilisierung der Azomethin-Bindung
5. S-300-Gelpermeationschromatografie

Mit dem erfindungsgemäßen Verfahren sind davon vor allem die Stufen 2 und 3 verknüpft, sie werden deshalb ausführlich am Beispiel der HRP-Markierung von IgG(Kaninchen) mit 1,13-Diamino-4,7,10-trioxa-tridecan beschrieben. Die Stufen 1, 4 und 5, die mit der Erfindung nur indirekt im Zusammenhang stestehen und allgemein bekannt sind, werden deshalb nur kurzgefaßt dargestellt.

### 1. HRP-Oxydation mit NalO4:

4,0 mg Meerrettichperoxidase (HRP, 100 nmol) in ein geeignetes Reaktionsröhrchen einwägen, in 0,8 ml Wasser lösen und 0,2 ml einer frisch hergestellten 0,1 M NalO₄-Lösung zu der kupferroten HRP-Lösung pipettieren, deren Farbe hierbei nach Schwarzgrün umschlägt. Das Reaktionsröhrchen sofort in eine verschließbare Hülse bringen, um Lichteinwirkung zu vermeiden ! Die Reaktionszeit beträgt 15 min bei Raumtemperatur. Gelegentlich das Röhrchen etwas bewegen.
Zur Beseitigung des NalO₄-überschusses werden 50 µl Ethylenglycol zugefügt. Auch diese Stopp-Reaktion wird im Dunkeln ausgeführt, die Reaktionszeit sollte wenigstens 30 min betragen. Die HRP(ox.)-Lösung ist danach wieder kupferrot gefärbt.
Die Abtrennung der HRP(ox.) von niedermolekularen Reaktionspartnern kann durch Gelfiltration im Ausschlußvolumen einer Sephadex-G-25-Säule (ca. 30 cm lang, 12 ml Gelbettvolumen) erfolgen. Es genügt, 20 Fraktionen zu je 0,5 ml abzunehmen. Die rotbraun gefärbten, HRP-haltigen Fraktionen werden gepoolt und durch Ultrafiltration (10000 NMGG) auf ein Volumen ≤ 0,5 ml eingeengt.

### 2. HRP(ox.)-Vernetzung mit DTT bei äguimolarem Ansatz:

In ein 50 ml-Maßkölbchen werden 10 µl DTT (δ ≈ 1,005) pipettiert, das Kölbchen wird mit 0,1 M Bicarbonatpuffer (pH 9,8) zur Marke aufgefüllt. Diese DTT-Lösung kann länger als 2 Monate verwendet werden.
Von dieser Lösung werden 0,1 ml (100 nmol) zu der eingeengten HRP(ox.)-Lösung pipettiert. Ein Mikrorührer wird zugefügt, mit dem ein rasches und gründliches Vermischen der Reaktionslösung ausgeführt werden kann. Die Reaktionszeit zur Vernetzung beträgt 4 Stunden bei Raumtemperatur; aus Zeitgründen kann das Reaktionsgemisch auch über Nacht in den Kühlschrank gestellt werden.

### 3. IgG-Kopplung mit vernetzter HRP:

Um einen hohen Substitutionsgrad zu erreichen, sollte das Stöchiometrieverhältnis HRP(vernetzt) zu IgG etwa 5 : 1 betragen. Unter Berücksichtigung eines geringen HRP-Verlustes bei den Präparationsstufen 1 und 2 werden zur Kopplung 2,25 bis 2,50 mg IgG(Kaninchen) = 15 bis 16,7 nmol eingesetzt. Das Immunglobulin wird entweder direkt in das Reaktionsröhrchen gegeben, das die vernetzte HRP-Lösung in 0,1 M Bicarbonatpuffer (pH 9,8) enthält, und durch Rühren in Lösung gebracht, oder zuvor in 0,1 M Bicarbonatpuffer (pH 9,8) gelöst und zugefügt. Die Reaktionsbedingungen bei der IgG-Kopplung sind die gleichen wie bei der Vernetzung: man läßt entweder 4 Stunden bei Raumtemperatur reagieren, oder stellt das Kopplungsgemisch über Nacht in den Kühlschrank. Es ist darauf zu achten, daß das Reaktionsvolumen 0,8 ml nicht übersteigt.

### 4. Stabilisierung der Azomethin-Bindung:

Zur Kopplungslösung werden zunächst 50 µl 2 M Triethanolamin (pH 8,0) gefügt, das Reaktionsgemisch wird gut durchgerührt und zur Abkühlung einige Minuten in den Kühlschrank gestellt. Zur Hydrierung der Azomethin-Bindungen wird eine 0,2 M NaBH₄-Lösung hergestellt, wozu B mg NaBH₄ erst unmittelbar vor Gebrauch in 1,0 ml kaltem Wasser gelöst werden. Von dieser Lösung werden nicht mehr als 75 µl der Kopplungslösung zugefügt. Beim Durchrühren schäumt die Reaktionslösung heftig auf. Die Reaktionszeit beträgt zunächst ca. 30 min im Kühlschrank, ehe noch einmal 25 µl 2 M Triethanolamin (pH 8,0) zugegeben werden. Das Reaktionsgemisch wird weitere 2 Stunden in den Kühlschrank gestellt. Schließlich werden zum Zwecke der Stabilisierung noch 10 µl 1 M Glycin (pH 7,0) zugesetzt.

### 5. S-300-Gelpermeationschromatografie:

Die Abtrennung des HRP-Tracers erfolgt auf einer 75 cm langen Sephacryl-S-300-Säule (ca. 41 ml Gelbettvolumen), die mit 0,05 M PBS-Puffer/0,15 M NaCl (pH 7,4) eluiert wird. Es werden 35 - 40 Fraktionen zu 1,0 ml abgenommen. Nurdie Fraktionen mit den hohen Extinktionen bei 402 nm, die in EIA die günstigsten Eigenschaften bezüglich ihrer spezifischen und unspezifischen Bindungen aufweisen, das sind meistens nur 3 Röhrchen, werden gepoolt.

### Anhang

| **Übersichtstabelle** | | |
|---|---|---|
| Zur Peroxidase-Vernetzung geeignete α,ω-Diamino-oxa-alkane | | |
| H₂N-CH₂-CH₂-O-CH₂-CH₂-O-CH₂-CH₂-NH₂ 1,8-Diamino-3,6-dioxa-octan | C₆H₁₆N₂O₂ | 148,2066 |
| H₂N-CH₂-CH₂-CH₂-O-CH₂-CH₂-CH₂-CH₂-O-CH₂--CH₂-CH₂-NH₂ 1,12-Diamino-4,9-dioxa-dodecan | C₁₀H₂₄N₂O | 204,3150 |
| H₂N-CH₂-CH₂-CH₂-O-CH₂-CH₂-O-CH₂-CH₂-O-CH₂-CH₂-CH₂-NH₂ 1,13-Diamino-4,7,10-trioxa-tridecan | C₁₀H₂₄N₂O₃ | 220,3144 |

## Patentansprüche

1. Verfahren zur enzymatischen Markierung von Biomolekülen, darunter Immunoglobuline, Peptide und Hormone oder auch Haptene, durch Kopplung mit Meerettichperoxidase (HRP), die zuvor in bekannter Weise mit Natriummetaperjodat oxydiert wird, **dadurch gekennzeichnet, dass** vor der Kopplung die oxydierte HRP mit einem längerkettigen α,ω-Diamino-oxa-alkan vernetzt wird.

2. Verfahren nach Anspruch 1, **dadurch gekennzeichnet, dass** zur Vernetzung 1,13-Diamino-4,7,10-trioxatridecan verwendet wird.

## Claims

1. Method for the enzymatic marking of biomolecules, amongst them immunoglobulines, peptides and hormones, or also haptens, by coupling with horseradish peroxidase (HRP), oxidised beforehand with sodium metaperiodate in a known way, wherein the oxidised HRP is linked with a longer-chained α,ω-diamino-oxa-alkane before the coupling.

2. Method according to claim 1, wherein 1,13-diamino-4,7,10-trioxatridecane is used for the linking.

## Revendications

1. Procédé de marquage enzymatique de biomolécules, dont font partie les immunoglobulines, les peptides et les hormones ou même les haptènes, par association avec des peroxydases du raifort (HRP) qui seront oxydées au préalable de la manière connue avec des métapériodates de sodium,
**se caractérisant par le fait qu'**avant l'association les HRP oxydés sont réticulés avec un α, ω diamino-oxa-alcane à plus longue chaîne;

2. Procédé selon la revendication 1,
**se caractérisant par le fait que** 1,13-diamino-4,7,10 -trioxatridecane est utilisé pour la réticulation;
